(19) Europäisches Patentamt
European Patent Office
Office européen des brevets

(11) Publication number : **0 325 482 B1**

(12) # EUROPEAN PATENT SPECIFICATION

(45) Date of publication of patent specification : **01.04.92 Bulletin 92/14**

(51) Int. Cl.$^5$ : **C07F 9/38,** A61K 31/66, C07F 9/40

(21) Application number : **89300560.3**

(22) Date of filing : **20.01.89**

(54) **(Cycloalkylamino)methylenebisphosphonic acids and medicines containing them.**

(30) Priority : **20.01.88 JP 11656/88**

(43) Date of publication of application : **26.07.89 Bulletin 89/30**

(45) Publication of the grant of the patent : **01.04.92 Bulletin 92/14**

(84) Designated Contracting States : **AT BE CH DE ES FR GB GR IT LI LU NL SE**

(56) References cited :
**EP-A- 0 224 751**
**DE-A- 2 037 586**
**US-A- 3 957 160**
**PATENT ABSTRACTS OF JAPAN vol. 3, no. 63 (C47), 30 May 1979 ; & JP-A-54 37829**

(73) Proprietor : **YAMANOUCHI PHARMACEUTICAL CO. LTD.**
**No. 3-11 Nihonbashi-Honcho, 2-chome**
**Chuo-ku**
**Tokyo (JP)**

(72) Inventor : **Isomura,Yasuo**
**Tabata sukai haitsu 4-12-11-919, Nishiogu**
**Arakawa-ku tokyo (JP)**
Inventor : **Takeuchi, Makoto**
**Purejiruenomoto 201 3-63-16, Higashi Oizumi**
**Nerima-ku Tokyo (JP)**
Inventor : **Sakamoto, Shuichi**
**Takagiyasaka manshion 402 3-17-7,**
**Sakae-cho**
**Higashimurayama-shi Tokyo (JP)**
Inventor : **Abe, Tetsushi**
**7-8-9, Azuma-cho**
**Iruma-shi Saitama (JP)**

(74) Representative : **Geering, Keith Edwin et al**
**REDDIE & GROSE 16 Theobalds Road**
**London WC1X 8PL (GB)**

EP 0 325 482 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to (cycloalkylamino)methylenebis phosphonic acids and lower alkyl esters and salts thereof which are useful as medicines having bone resorption inhibitory, anti-inflammatory, and antirheumatic effects.

Some (cycloalkylamino)methylenebis phosphonic acids are known. JP laid-open Application No. 37,829/79 and JP Patent Publication No. 12,319/80 disclose the unsubstituted cyclopentyl and cyclohexyl compounds.

These disclosures mention these acids as agricultural chemicals, especially herbicides, and for preventing precipitation in water or aqueous solution, but do not suggest their use as medicines.

The present invention provides a pharmaceutical composition containing active ingredient selected from (cycloalkylamino)methylenebisphosphonic acids and esters (I)

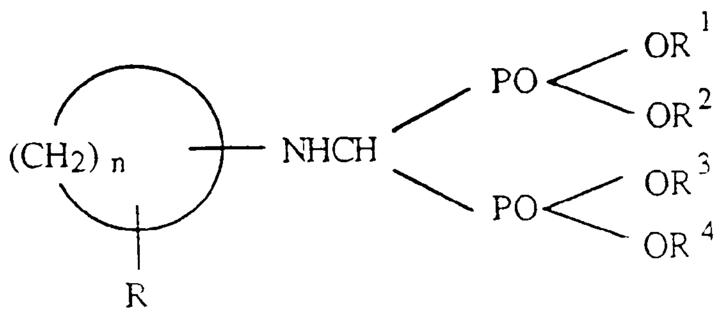

(I)

and pharmaceutically acceptable salts thereof, in which R, $R^1$, $R^2$, $R^3$ and $R^4$ are selected independently from a hydrogen atom and lower alkyl groups, and n is an integer from 3 to 10.

The present invention also provides a bone-resorption inhibitor and/or anti-arthritic containing active ingredient selected from these compounds.

It also provides novel acids and esters (I) and salts thereof, in which R, $R^1$, $R^2$, $R^3$, $R^4$ and n are as defined above except that R is lower alkyl when n is 5 or 6 and $R^1$, $R^2$, $R^3$ and $R^4$ are H.

Herein, "lower alkyl" is a linear or branched group having 1 to 5 carbon atoms, e.g. a methyl, ethyl, propyl or isopropyl group.

The group represented by chemical structure

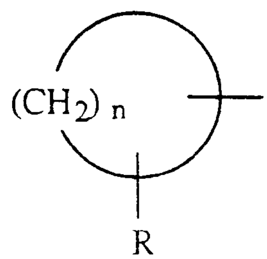

herein is a cyclopropyl, cyclobutyl, cyclopentyl, cyclohexyl, cycloheptyl, cyclooctyl, cyclononyl or cyclodecyl group optionally substituted by lower alkyl.

Compounds (I) form salts with bases;preferred pharmaceutically acceptable salts are those with inorganic bases such as sodium and potassium salts and the like or with organic bases such as ammonium and triethylamine salts and the like.

Compounds used in the present invention can be prepared as follows :

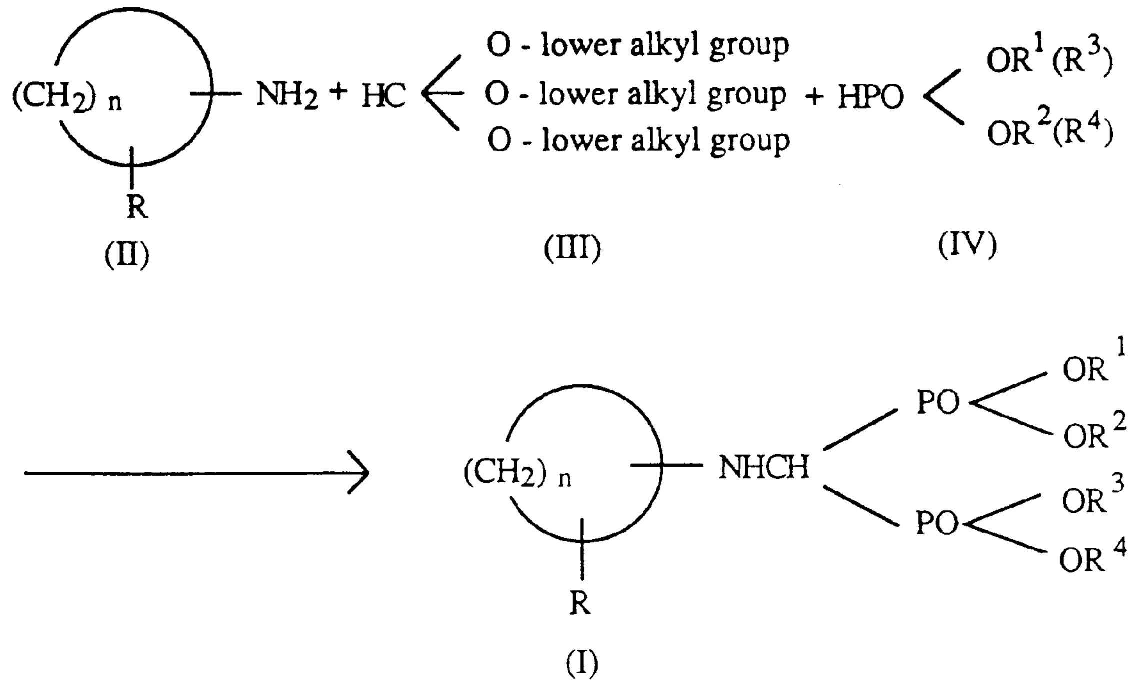

(II) (III) (IV)

(I)

In this reaction cycloalkylamine (II), lower alkyl ortho-formate (III) and phosphorous acid and/or lower alkyl ester(s) (IV) are mixed in the corresponding reaction proportions and heated. A reaction solvent is not specifically required. The reaction is performed usually at 100 to 200°C, preferably at 150°C or so, for 10 to 60 minutes.

For isolating and purifying the reaction product, for example, the reaction mixture is charged to a silica gel column and eluted with a mixed solvent of methanol-chloroform. In the above reaction, the corresponding bisphosphonic acid or bisphosphonate can be obtained respectively from phosphorous acid or ester(s) (IV).

The bisphosphonates can be converted into the corresponding bisphosphonic acids by hydrolysis. The hydrolysis is generally carried out by heating under reflux in concentrated hydrochloric acid. Alternatively, the bisphosphonates can be treated with strong acid or trimethylsilyl halide in a water-free solvent;

commercial anhydrous hydrobromic acid in acetic acid can be used directly or as an appropriately diluted solution, or a solution of trimethylsilyl iodide in a solvent such as carbon tetrachloride, dimethylformamide, chloroform, toluene, etc. can be used. The hydrolysis can be carried out with cooling or heating. For example, when the ester is hydrolyzed with trimethylsilyl halide with cooling at -10°C or lower, a partially hydrolyzed product is obtained.

When a bisphosphonic acid is to be converted into its salt, it can be treated with a base such as sodium hydroxide, potassium hydroxide, ammonia or organic amines, etc. in conventional manner.

The compounds (I) and their salts have bone resorption-inhibitory action and inhibit hypercalcemia caused by bone resorption. In addition they have anti-inflammatory action and antipyretic and analgesic action.

Experimental test methods and results below demonstrate the medicinal activity of compositions and compounds according to the invention.

(1) Inhibitory Effect on Hypercalcemia of Rats

Rats with hypercalcemia induced by administration of parathyroid hormone were used, and the decrease in serum calcium due to administration of test compound was measured. Control groups of rats received the hormone but no test compound, and normal control groups no hormone and no test compound.

Test Method

Human 1-34 parathyroid hormone, (PTH, manufactured by Peptide Laboratory) dissolved in 0.1 % BSA (bovine serum albumin)-containing physiological saline (PTH content 6 μg/ml) was intravenously injected at 30 μg/kg (5 ml of solution/kg) into 5-week male Wistar rats which had been fasting for 20 hours; the BSA-containing saline alone (no PTH) was injected into normal control groups in the same manner. 45 minutes after

the BSA injection, the rats were etherized and then subjected to celiotomy, whereby the blood was collected from the abdominal cava with a vacuum blood-collecting tube. The blood collected was immediately centrifuged at 3000 rpm at 4 °C for 10 minutes to isolate the serum. The ionized calcium ($Ca^{++}$) concentration in the serum was immediately measured with a $Ca^{++}$ meter (Sera 250, manufactured by Horiba Manufacturing Co.).

Test compounds dissolved using sodium hydroxide and hydrochloric acid, in physiological saline (pH 7.4) for subcutaneous administration, were administered to respective test groups in doses of 2 ml/kg 72 hours before the PTH injection. As a reference compound, salmon calcitonin (SCT, manufactured by Armour Co.) was used. The SCT was subcutaneously administered at a dose of 2 ml/kg 30 minutes before the PTH injection.

The results for each group were expressed in terms of mean ± S.E. (standard error) and comparison was made among the groups by testing by one-way analysis of variance. The significance level was taken at 5%. In the Tables below, N is the number of rats in the group concerned.

Results

The results obtained by subcutaneous administration are shown in Table 1.

Table 1 Subcutaneous administration

| Compound Tested | Dose (mg/kg) | N | Serum $Ca^{++}$ (m mole / ℓ) |
|---|---|---|---|
| Normal control (-PTH) | - | 5 | 1.42 ± 0.02 |
| Control (+PTH) | - | 5 | 1.48 ± 0.03 |
| Compound of Example 8 | 0.3 | 5 | 1.25 ± 0.02** |
| Compound of Example 8 | 1.5 | 5 | 1.12 ± 0.02** |
| Normal Control (-PTH) | - | 5 | 1.41 ± 0.02** |
| Control (+PTH) | - | 5 | 1.46 ± 0.02 |
| Compound of Example 15 | 0.03 | 5 | 1.37 ± 0.02** |
| Compound of Example 15 | 0.1 | 5 | 1.20 ± 0.02** |

Table 1 (continued)

| Compound Tested | Dose (mg/kg) | N | Serum $Ca^{++}$ (m mole / ℓ) |
|---|---|---|---|
| Normal control (-PTH) | - | 5 | 1.34 ± 0.02** |
| Control (+PTH) | - | 5 | 1.43 ± 0.01 |
| Compound of Example 14 | 0.1 | 5 | 1.34 ± 0.02** |
| Compound of Example 14 | 0.3 | 5 | 1.21 ± 0.01** |
| Compound of Example 5 | 0.1 | 5 | 1.12 ± 0.01** |
| Compound of Example 5 | 0.3 | 5 | 0.97 ± 0.02** |
| Normal Control (-PTH) | - | 5 | 1.36 ± 0.01** |
| Control (+PTH) | - | 5 | 1.45 ± 0.02 |
| Compound of Example 9 | 0.1 | 5 | 1.31 ± 0.01** |
| Compound of Example 9 | 0.3 | 5 | 1.19 ± 0.01** |
| Compound of Example 10 | 0.1 | 5 | 1.35 ± 0.01** |
| Compound of Example 10 | 0.3 | 5 | 1.22 ± 0.01** |
| Normal control (-PTH) | - | 5 | 1.35 ± 0.02** |
| Control (+PTH) | - | 5 | 1.44 ± 0.01 |
| Compound of Example 11 | 1.0 | 5 | 1.35 ± 0.02** |
| Normal control | - | 5 | 1.38 ± 0.01** |
| Control | - | 5 | 1.48 ± 0.02 |
| Compound of Example 7 | 0.3 | 5 | 1.40 ± 0.02** |
| Compound of Example 7 | 1.0 | 5 | 1.29 ± 0.01** |
| Compound of Example 6 | 0.3 | 5 | 1.33 ± 0.03** |
| Compound of Example 6 | 1.0 | 5 | 1.12 ± 0.03** |
| Normal control (-PTH) | - | 5 | 1.38 ± 0.01** |
| Control (+PTH) | - | 5 | 1.49 ± 0.00 |
| SCT | 0.3 IU | 5 | 1.07 ± 0.02** |

Note: Mean value ± S. E. * : $P<0.05$ ** : $P<0.01$

(2) PTH-Induced Hypercalcemia in Rats

Method

PTH (human PTH 1-34, 30 μg/kg) was intravenously injected into rats (Wistar, male, about 3-week-old). Blood was collected 45 min. after PTH injection. Ionized calcium ($Ca^{++}$) in the serum was measured with a $Ca^{++}$ meter.

Test compounds were subcutaneously or orally administered three days before PTH injection. Results are expressed as mean ±S.E. Statistical significance of the values was analyzed using One-Way ANOVA test (*:

p<0.05, **:p<0.01). The test compounds were dissolved in physiological saline for subcutaneous administration, and in distilled water for oral administration. Control (+PTH) and normal (-PTH) groups received no test compound.

Results

PTH elevated serum $Ca^{++}$ level,probably by stimulation of calcium release from the bone. Bisphosphonates (the compound of Example 5 (hereinafter, YM-21175-1) and APD*) dose-dependently inhibited the increase of serum $Ca^{++}$ level when subcutaneously or orally administered to the rats 3 days before PTH injection. YM-21175-1 was about 10 times more potent than APD in both subcutaneous and oral administrations.

$$\text{APD (Ciba-Geigy) is } H_2NCH_2CH_2\underset{\displaystyle OH}{\underset{|}{C}}(PO_3H_2)_2\ ,$$

a commercial medicine for treating Paget's disease.

Table 2.

Effects of YM-21175-1 and APD on PTH-induced hypercalcemia in rats

| | Dose (mg/kg) | | N | Serum $Ca^{++}$ (m mole/ℓ) |
|---|---|---|---|---|
| normal (-PTH) | - | | 5 | 1.42 ± 0.02 |
| control (+PTH) | - | | 5 | 1.49 ± 0.02 |
| A P D | 0.03 | sc | 5 | 1.49 ± 0.02 |
| | 0.1 | sc | 5 | 1.46 ± 0.01 |
| | 0.3 | sc | 5 | 1.41 ± 0.02* |
| A P D | 30 | po | 5 | 1.50 ± 0.02 |
| | 100 | po | 5 | 1.42 ± 0.02 |
| | 300 | po | 5 | 1.22 ± 0.02** |
| YM-21175-1 | 0.01 | sc | 5 | 1.44 ± 0.02 |
| | 0.03 | sc | 5 | 1.36 ± 0.02** |
| | 0.1 | sc | 5 | 1.15 ± 0.01** |
| normal (-PTH) | - | | 5 | 1.35 ± 0.02 |
| control (+PTH) | - | | 5 | 1.45 ± 0.02 |
| YM-21175-1 | 10 | po | 5 | 1.37 ± 0.02 |
| | 30 | po | 5 | 1.23 ± 0.05** |
| | 100 | po | 5 | 1.05 ± 0.04** |

(3) Disuse Atrophy of Bone Induced by Neurectomy in Rats

Method

Rats (Wistar, male, 6-week-old) were neurectomized at the brachial plexisus so that their left forelimbs became disused. A normal group was not neurectomized. Test compounds were orally administered once a day for 2 weeks following neurectomy; a control group received no test compound. 24 hours after the final administration, the left humerus was removed from each rat in the normal, control and test groups. The soft tissue around the bone was cleaned off, the bone was dehydrated and defatted with alcohol and acetone successively, and the dry weight of the bone was measured. Results were expressed as mean ±S. E. Statistical significance of the values was analyzed using One-Way ANOVA test (*: $p<0.05$, **: $p<0.01$).

Results

The dry weight of the denerved humerus was significantly decreased as compared with that of the normal one. Oral administration of bisphosphonates YM-21175-1 and APD demonstrated dose-dependent inhibition of dry weight loss in the denerved humerus compared to the control.
YM-21175-1 was about 30 times superior to APD.

Table 3.

Effects of YM-21175-1 and APD on disuse atrophy of bone induced by neurectomy in rats

| | Dose (mg/kg) | N | dry wt (mg) |
|---|---|---|---|
| Normal | - | 6 | 154 ± 3** |
| control | - | 6 | 113 ± 2 |
| A P D | 10 | 5 | 126 ± 3 |
| | 30 | 5 | 128 ± 4* |
| | 100 | 6 | 135 ± 2** |
| YM-21175-1 | 0.3 | 6 | 126 ± 2 |
| | 1 | 6 | 132 ± 6** |
| | 3 | 6 | 135 ± 3** |
| | 10 | 6 | 150 ± 4** |

(4) Adjuvant-Induced Arthritis in Rats

Method

A suspension of dead bacilli in oil was intradermally injected into left hind paws of rats (Lewis, male, 5-week-old). Compounds were orally given daily for 5 weeks, starting from the day of injection of the adjuvant. Left hind foot thickness was measured and the left femurs were removed the day after last dosing. The bones were dehydrated and defatted, and the dry weight measured; the ash weight was then measured. A control group received no test compound, and a normal group no bacilli and no test compound.

Bone mineral content (BMC) was calculated as ash wt/dry wt. Results were expressed as mean ±S.E. (N=6). Statistical significance of the values was analyzed using One-Way ANOVA test (**:$p<0.01$).

Results

Adjuvant-induced arthritis is a popular model for rheumatoid arthritis in humans.Arthritic rats induced by injection of the adjuvant show not only swelling of the foot but also decrease of BMC, which is supposed to be caused by increase of bone resorption and/or disuse of hindlimbs.

Indomethacin (1 mg/kg) markedly inhibited the swelling of hindlimbs. It also inhibited the decrease of BMC as a result of the inhibition of development of arthritis. YM-21175-1 inhibited the swelling only at high doses but decrease of BMC at 1 mg/kg. Therefore there is probably a difference in inhibitory action between indomethacin and YM-21175-1. YM-21175-1 was more effective than APD.

Table 4.

Effects of YM-21175-1 and APD on adjuvant-induced arthritis in rats

| | dose (mg/kg) | N | thickness (mm) | ash wt/dry wt (%) |
|---|---|---|---|---|
| normal | - | 6 | 6.4 ± 0.0 | 54.8 ± 0.1** |
| control | - | 6 | 13.5 ± 0.1 | 49.5 ± 0.4 |
| indomethacin | 1 | 6 | 7.6 ± 0.1** | 52.3 ± 0.2** |
| A P D | 1 | 6 | 12.1 ± 0.6 | 50.4 ± 0.3 |
| | 3 | 6 | 11.6 ± 0.7 | 51.2 ± 0.4** |
| | 10 | 6 | 10.8 ± 0.6** | 52.1 ± 0.5** |
| | 30 | 6 | 12.1 ± 0.3 | 53.3 ± 0.3** |
| | 100 | 6 | 9.3 ± 0.3** | 56.6 ± 0.3** |
| YM-21175-1 | 0.3 | 6 | 12.3 ± 0.6 | 50.7 ± 0.6 |
| | 1 | 6 | 11.7 ± 0.1 | 52.1 ± 0.8** |
| | 3 | 6 | 12.3 ± 0.8 | 53.1 ± 0.4** |
| | 10 | 6 | 10.5 ± 0.7** | 55.9 ± 0.3** |
| | 30 | 5 | 9.5 ± 0.2** | 57.6 ± 0.3** |

The inhibitory effect of YM-21175-1 on bone loss was compared with APD. From three kinds of experiment, YM-21175-1 seems to be useful in the treatment of osteoporosis, rheumatoid arthritis and other diseases in which bone resorption is accelerated, and besides YM-21175-1 is concluded to be more potent that APD.

From the test results, the compounds for the present invention are seen to have marked action for reducing the amount of serum calcium and to have bone-resorption inhibitory action. As diseases considered to be caused by excessive bone-resorption, there may be mentioned Paget's disease, hypercalcemia, metastatic osteocarcinoma, and osteopsathrosis. Further, sthenic bone resorption to follow inflammatory arthritides such as chronic rheumatoid arthritis is an important problem from a clinical point of view. The compositions provided by the present invention can be used as remedial medicines for these diseases to inhibit bone resorption, prevent the reduction of bone, or decrease or prevent increase of the serum calcium value caused by sthenic bone resorption.

The compounds for the present invention can be blended with any optional pharmaceutically acceptable

carrier, vehicle, attenuant, etc. to be formed into medical compositions - e.g. tablets, capsules, powder, granules, pills, etc. for oral administration, or injections, syrups, suppositories, ointments etc. for non-oral administration. The dose, though varying with administration route, patient's symptoms etc., is generally from 1 mg/day/adult to 1 g/day/adult for oral administration, and from 0.1 to 10 mg/day/adult for non-oral administration.

Prescription Examples

(1) Tablet:

| | |
|---|---|
| Compound of Example 8 | 5 mg |
| Lactose | 119 mg |
| Corn Starch | 67 mg |
| Hydroxypropyl Cellulose | 4 mg |
| Calcium Carboxymethyl Cellulose | 4 mg |
| Magnesium Stearate | 1 mg |
| Total | 200 mg |

5 g of the compound of Example 8, 119 g of lactose and 67 g of corn starch were uniformly blended, 40 ml of an aqueous 10 % (w/w) hydroxypropyl cellulose solution was added thereto, and the resulting mixture was wet-granulated. The granules thus obtained were blended with 4 g of calcium carboxymethyl cellulose and 1 g of magnesium stearate, and the resulting mixture formed into 200 mg.tablets.

(2) Capsule:

| | |
|---|---|
| Compound of Example 8 | 5 mg |
| Crystalline Cellulose | 50 mg |
| Crystalline lactose | 144 mg |
| Magnesium Stearate | 1 mg |
| Total | 200 mg |

The above-mentioned ingredients were blended in amounts 1000 times those above and encapsulated in gelatin capsules each containing 200 mg of the mixture.

## Preparation Examples

### Example 1

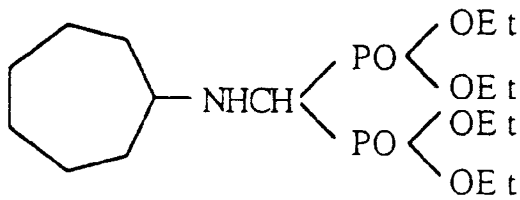

A mixture of 4.0 g of cycloheptylamine, 6.27 g of ethyl ortho-formate and 19.5 g of diethylphosphite was heated at 150 °C with stirring for 1.5 hours. After cooling, the reaction solution was concentrated under reduced pressure to eliminate ethyl ortho-formate and diethylphosphite which were not reacted. The residue was purified by silica gel column chromatography (methanol/chloroform = 1/49) to give 9.0 g of tetraethyl (cycloheptylamino)methylenebis(phosphonate) as a pale yellow oil.

The physico-chemical characteristics of this product are as follows:

(i) Mass Spectrum (FAB Mass) 400 ($M^+$ + 1)

(ii) Nuclear Magnetic Resonance Spectrum (δ value, in $CDCl_3$)

1.32 (12H, $OCH_2C\underline{H}_3$ x 4)

1.20 ~ 2.08 (12H, H methylene in cycloheptyl group)

2.96 (1H, 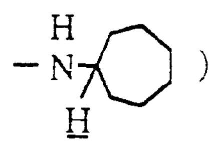 )

3.36 (1H, -NHC$\underline{H}$-)

4.00 ~ 4.40 (8H, -$OC\underline{H_2}CH_3$ × 4)

In the same manner as Example 1, the following compounds were prepared.

### Example 2

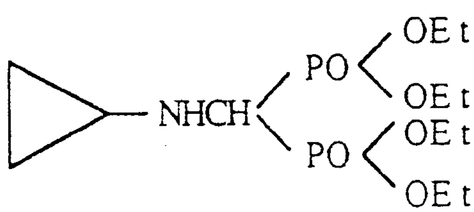

Tetraethyl (cyclopropylamino)methylenebis(phosphonate)

Yellow oil

(i) Mass Spectrum (FAB Mass) 344 ($M^+$ + 1)

(ii) Nuclear Magnetic Resonance Spectrum (δ value, in $CDCl_3$)

0.36 ~ 0.56 (4H. 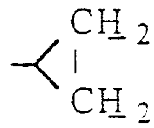 )

1.35 (12H, $OCH_2C\underline{H}_3 \times 4$)

1.94 (1H, -N$\underline{H}$-)

2.65 (1H 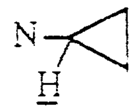 )

3.40 (1H, -NC$\underline{H}$-)

3.96 ~ 4.40 (8H, $-OC\underline{H}_2CH_3 \times 4$)

Example 3

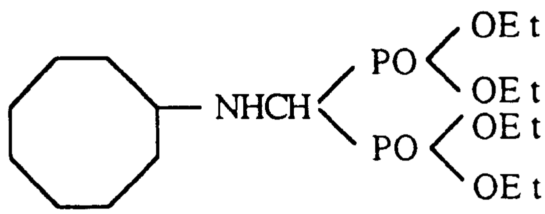

Tetraethyl(cyclooctylamino)methylenebis(phosphonic acid)

(i) Mass Spectrum (FAB Mass) 414 ($M^+ + 1$)

(ii) Nuclear Magnetic Resonance Spectrum (δ value, in $CDCl_3$)

1.34 (12H, $-OCH_2C\underline{H}_3 \times 4$)

1.20 ~ 2.40 (14H, H methylene in cyclooctyl group)

3.04 (1H, 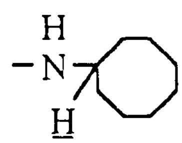 )

3.36 (1H, -NHC$\underline{H}$-)

4.00 ~ 4.48 (8H, $-OC\underline{H}_2CH_3 \times 4$)

Example 4

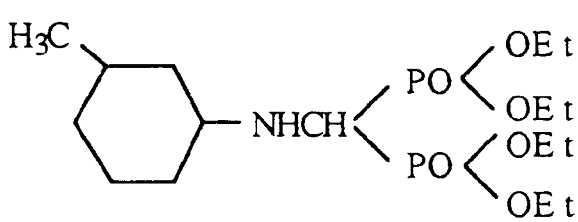

Tetraethyl[(3-methylcyclohexyl)amino]methylenebis(phosphonate)

(i) Mass Spectrum (FAB Mass) 400 ($M^+ + 1$)

(ii) Nuclear Magnetic Resonance Spectrum

0.92 (3H, 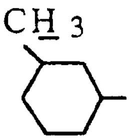 )

1.34 (12H, $-OCH_2C\underline{H}_3 \times 4$)

1.20 ~ 2.40 (9H, 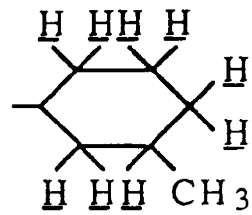 )

2.80 (1H, 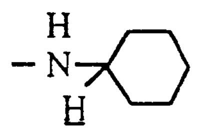 )

3.44 (1H, $-NC\underline{H}-$)

4.00 ~ 4.42 (8H, $-OC\underline{H}_2CH_3 \times 4$)

Example 5

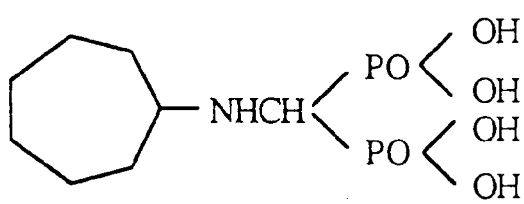

4.0 g of tetraethyl (cycloheptylamino)methylenebis(phosphonate) was dissolved in 40 ml of concentrated hydrochloric acid and heated under reflux for 2.5 hours. After cooling, the reaction solution was concentrated under reduced pressure to eliminate hydrochloric acid. Then, 30 ml of purified water was added to the residue and the mixture was again concentrated under reduced pressure. The oily product thus obtained was precipitated by methanol and acetone and filtered. The residue was washed with acetone to give 2.5 g of (cycloheptylamino)methylenebis(phosphonic acid) as a white solid.

This had the following physico-chemical properties:

(i) Mass Spectrum (FAB Mass) 288 ($M^+ + 1$)

(ii) Elementary Analysis ($C_8H_{19}NO_6P_2$)

| | C | H | N | P |
|---|---|---|---|---|
| Calculated (%): | 33.46 | 6.67 | 4.88 | 21.57 |
| Found (%): | 33.27 | 6.40 | 4.87 | 21.54 |

(iii) m.p.:232 to 233 °C (Recrystallization from Me OH-$H_2O$)

In the same manner as Example 5, the following compounds were prepared.

Example 6

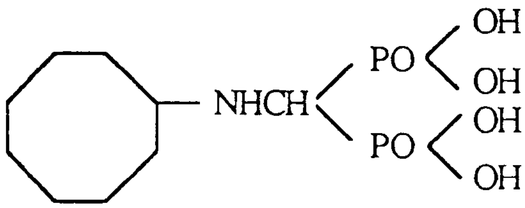

Cyclooctylamino)methylenebis(phosphonic acid)
(i) Mass Spectrum (FAB Mass) 302 ($M^+ + 1$)
(ii) Elementary Analysis ($C_9H_{21}NO_6P_2$)

| | C | H | N | P |
|---|---|---|---|---|
| Calculated (%): | 35.89 | 7.03 | 4.65 | 20.57 |
| Found (%): | 35.87 | 6.82 | 4.69 | 20.49 |

(iii) m.p. (°C) 228 to 229 (Not purified)

Example 7

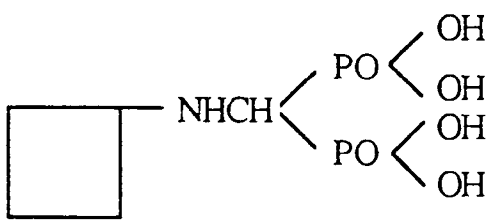

(Cyclobutylamino)methylenebis(phosphonic acid)
(i) Elementary Analysis ($C_5H_{13}NO_6P_2$)

| | C | H | N |
|---|---|---|---|
| Calculated (%): | 24.50 | 5.35 | 5.71 |
| Found (%): | 24.41 | 5.23 | 5.66 |

(ii) m.p. (°C) 256 to 258 (Recrystallization from methanol)

Example 8

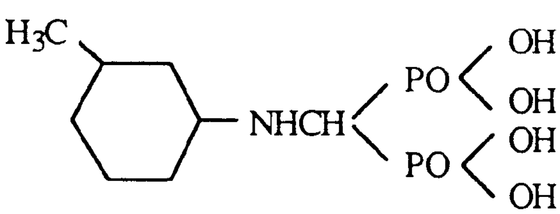

(cis.trans mixture)

[(3-methylcyclohexyl)amino]methylenebis(phosphonic acid)

(i) Mass Spectrum (FAB Mass) 288 ($M^+ + 1$)
(ii) Elementary Analysis ($C_8H_{19}NO_6P_2 \cdot 0.2H_2O$)]

| | C | H | N | P |
|---|---|---|---|---|
| Calculated (%): | 33.04 | 6.72 | 4.81 | 21.30 |
| Found (%): | 32.88 | 6.47 | 4.77 | 21.32 |

(iii) m.p. (°C) 220 to 221 (Not purified)

Example 9

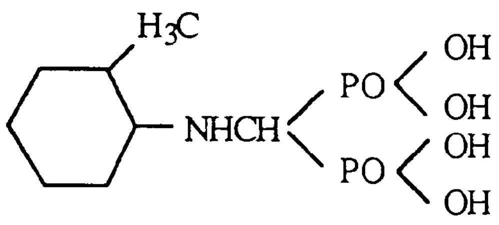

(cis.trans mixture)

[(2-methylcyclohexyl)amino]methylenebis(phosphonic acid)
(i) Elementary Analysis ($C_8H_{19}NO_6P_2$)

| | C | H | N |
|---|---|---|---|
| Calculated (%): | 33.46 | 6.67 | 4.88 |
| Found (%): | 33.07 | 6.39 | 4.86 |

(ii) m.p. (°C) 238 to 240 (Recrystallization from methanol-acetone)

Example 10

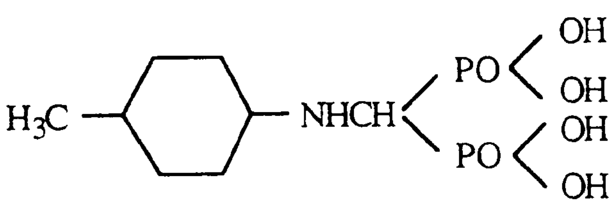

(cis.trans mixture)

[(4-methylcyclohexyl)amino]methylenebis(phosphonic acid)
(i) Elementary Analysis ($C_8H_{19}NO_6P_2$)

| | C | H | N |
|---|---|---|---|
| Calculated (%): | 33.46 | 6.67 | 4.88 |
| Found (%): | 33.13 | 6.41 | 4.75 |

(ii) m.p. (°C) 255 to 258 (Recrystallization from methanol-water)

Example 11

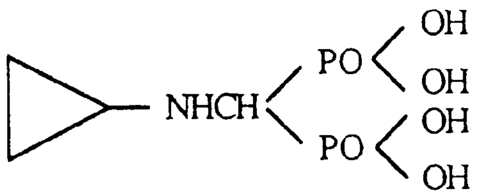

1.28 g of tetraethyl (cyclopropylamino)methylenebis(phosphonate) was dissolved in 13 ml of 25 % hydrogen bromide acetic acid solution and the mixture was stirred at 45 °C for 2 hours. The reaction solution was concentrated under reduced pressure, and 20 ml of purified water was added to the residue. Then, the mixture was again concentrated under reduced pressure. The oily product thus obtained was precipitated by methanol and acetone and subjected to filtration. The solid was washed with acetone to give 0.42 g of (cyclopropylamino)methylenebis(phosphonic acid) as a white solid.

The physico-chemical characteristics of this product are as follows:

(i) Mass Spectrum (FAB Mass) 232 ($M^+$ + 1)

(ii) Elementary Analysis ($C_4H_{11}NO_6P_2 \cdot 0.2\ H_2O$)

| | C | H | N | P |
|---|---|---|---|---|
| Calculated (%): | 20.47 | 4.89 | 5.96 | 26.39 |
| Found (%): | 20.45 | 4.73 | 5.83 | 26.33 |

(iii) m.p. (°C) 214 to 216 °C (Not purified)

Example 12

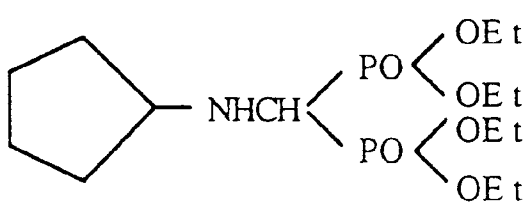

A mixed solution of 3.0 g of cyclopentylamine, 6.2 g of ethyl ortho-formate and 19.4 g of diethylphosphite was heated at 150 °C with stirring for 1.5 hours. After cooling, the reaction solution was concentrated under reduced pressure to eliminate ethyl ortho-formate and diethylphosphite which were not reacted. The residue was purified by silica gel column chromatography (methanol/chloroform = 1/49) to give 10.7 g of tetraethyl (cyclopentylamino)methylenebis-(phosphonate) as a pale yellow oil.

The pysico-chemical properties of this product are as follows:

(i) Mass Spectrum (FAB Mass): 372 ($M^+$ + 1)

(ii) Nuclear Magnetic Resonance Spectrum (δ value, in $CDCl_3$)

1.34 (12H, $OCH_2C\underline{H}_3 \times 4$)

1.42 ∼ 2.00 (8H, H methylene cyclopentyl group)

3.30 (1H, -NHC$\underline{H}$-)

3.48 (1H, 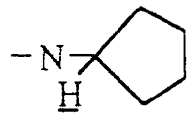 )

4.00 ∼ 4.36 (8H, $-OC\underline{H}_2CH3 \times 4$)

In the same manner as Example 12, the following compounds were prepared.

Example 13

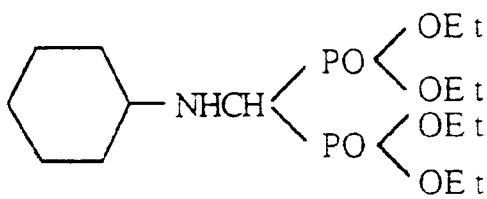

Tetraethyl(cyclohexylamino)methylenebis(phosphonate)
(i) Mass spectrum (FAB Mass) 386 ($M^+ + 1$)
(ii) Nuclear Magnetic Resonance Spectrum (δ value in $CDCl_3$)

1.32 (12H, $-OCH_2C\underline{H}_3$ x 4)

1.2 ∼ 2.0 (10H, H methylene in a cyclohexyl group)

2.90 (1H, 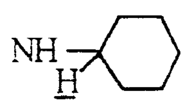 )

3.44 (1H, NHC$\underline{H}$)

4.00 ∼ 4.40 (8H, $-OC\underline{H}_2CH_3$ x 4)

Example 14

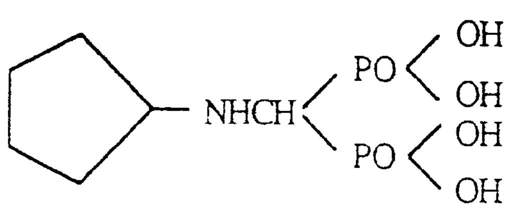

8.0 g of tetraethyl (cyclopentyl amino)methylenebis(phosphonate) was dissolved in 80 ml of concentrated hydrochloric acid and the mixture was heated under reflux for 2.5 hours. After cooling, the reaction solution was concentrated under reduced pressure to eliminate hydrochloric acid. 70 ml of purified water was added to the residue and the mixture was again concentrated under reduced pressure. The obtained product in the form

of oil was precipitated by acetone and acetonitrile and subjected to filtration. The product was recrystallized from water-methanol to give 3.6 g of (cyclopenthylamino)methylenebis(phosphonic acid) as white crystals.

The physico-chemical properties of this product are as follows:

(i) Mass Spectrum (FAB Mass): 260 ($M^+$ + 1)

(ii) Elementary Analysis ($C_6H_{15}NO_6P_2 \cdot 0.1\ H_2O$)

| | C | H | N | P |
|---|---|---|---|---|
| Calculated (%): | 27.62 | 5.87 | 5.37 | 23.74 |
| Found (%): | 27.42 | 5.67 | 5.48 | 23.66 |

(iii) m. p. (°C) 228 to 229 °C

In the same manner as Example 14 the following compound was prepared.

Example 15

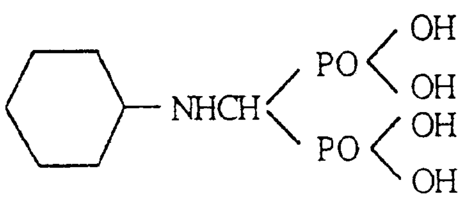

(Cyclohexylamino)methylenebis(phosphonic acid)

(i) Mass Spectrum (FAB Mass) 274 ($M^+$ + 1)

(ii) Elementary Analysis ($C_7H_{17}NO_6P_2$)

| | C | H | N | P |
|---|---|---|---|---|
| Calculated (%): | 30.78 | 6.27 | 5.13 | 22.68 |
| Found (%): | 30.48 | 6.11 | 5.16 | 22.17 |

(iii) m. p. (°C) 267 to 269 °C (Not purified)

**Claims**

**Claims for the following Contracting States : AT, BE, CH, DE, FR, GB, IT, LI, LU, NL, SE**

1. A pharmaceutical composition containing active ingredient selected from (cycloalkylamino)methylenebis phosphonic acids and esters (I) and pharmaceutically acceptable salts thereof

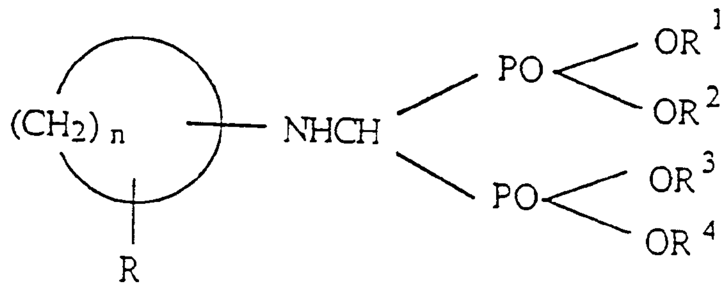

(I)

wherein R, $R^1$, $R^2$, $R^3$ and $R^4$ are selected independently from a hydrogen atom and ($C_1$-$C_5$ alkyl) groups and n is an integer from 3 to 10.

2. A composition according to claim 1 wherein active ingredient comprises (cycloheptylamino)methylene-bis(phosphonic acid) or ($C_1$-$C_5$ alkyl) ester or salt thereof.

3. A bone-resorption inhibitor composition according to claim 1 or 2.

4. An anti-arthritic composition according to claim 1 or 2.

5. (Cycloalkylamino)methylenebis(phosphonic acid) or ester (I) or salt thereof

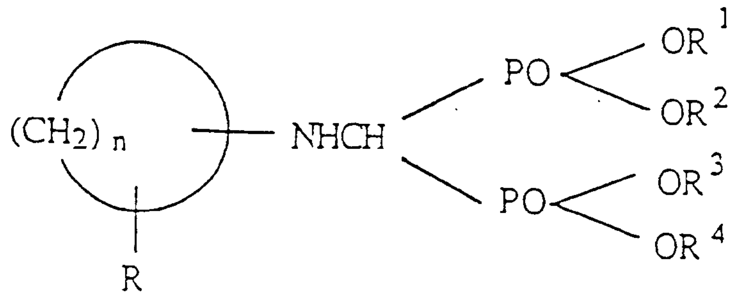

(I)

wherein R, $R^1$, $R^2$, $R^3$ and $R^4$ are selected independently from a hydrogen atom and ($C_1$-$C_5$ alkyl) groups and n is an integer from 3 to 10, with the proviso that R is ($C_1$-$C_5$alkyl) when $R^1$, $R^2$, $R^3$ and $R^4$ are hydrogen atoms and n is 5 or 6.

6. A compound according to claim 5 which is (Cycloheptylamino)methylenebis(phosphonic acid) or a ($C_1$-$C_5$ alkyl) ester or salt thereof.

7. A method of preparing a compound as defined in claim 5 which comprises reacting (a) the corresponding cycloalkylamine, (b) phosphorous acid and/or the corresponding ($C_1$-$C_5$) alkyl ester(s) thereof, and (c) ($C_1$-$C_5$ alkyl) orthoformate, and optionally converting the product to or from acid, ester or salt form.

**Claims for the following Contracting States : ES, GR**

1. A method of preparing a compound which is a (cycloalkylamino)methylenebis(phosphonic acid) or ester (I) or salt thereof

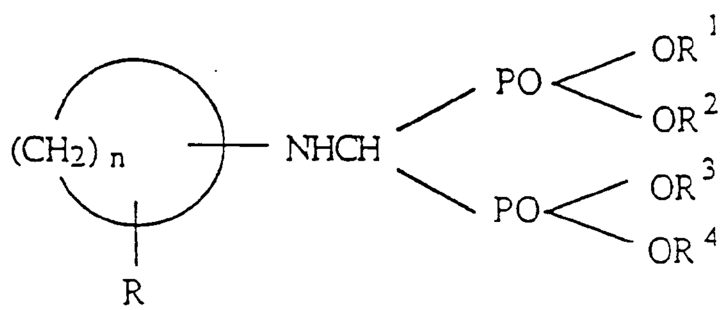

(I)

wherein R, $R^1$, $R^2$, $R^3$ and $R^4$ are selected independently from a hydrogen atom and ($C_1$-$C_5$ alkyl) groups and n is an integer from 3 to 10, with the proviso that R is ($C_1$-$C_5$ alkyl) when $R^1$, $R^2$, $R^3$ and $R^4$ are hydrogen atoms and n is 5 or 6, which comprises reacting (a) the corresponding cycloalkylamine

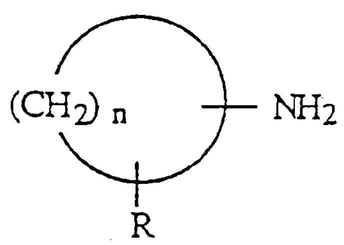

(b) phosphorous acid and/or the corresponding ($C_1$-$C_5$) alkyl ester(s) thereof

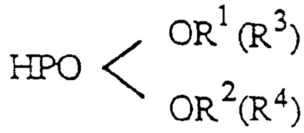

and (c) ($C_1$-$C_5$ alkyl) orthoformate

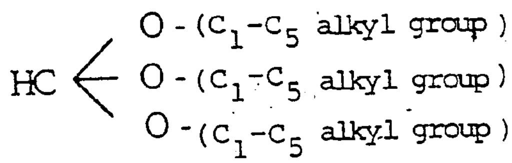

and optionally converting the product to or from acid, ester or salt form.

2. A method according to claim 1 wherein the product is (cycloheptylamino)methylenebis(phosphonic acid) or a ($C_1$-$C_5$ alkyl) ester or salt thereof.

## Patentansprüche

### Patentansprüche für folgende Vertragsstaaten : AT, BE, CH, DE, FR, GB, IT, LI, LU, NL, SE

1. Pharmazeutisches Mittel, enthaltend einen Wirkstoff, aus- gewählt aus (Cycloalkylamino)methylen-bis-phosphonsäuren und -estern (I) und pharmazeutisch annehmbaren Salzen davon

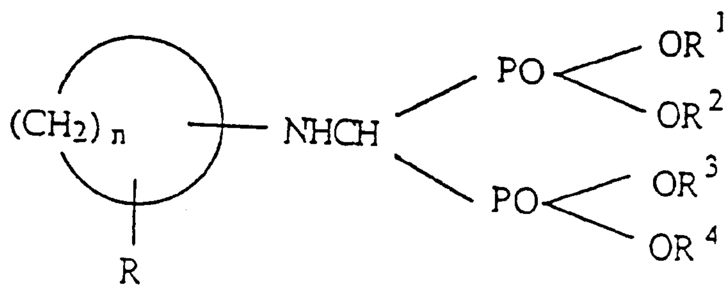

(I)

wobei R, $R^1$, $R^2$, $R^3$ und $R^4$ unabhängig voneinander ausgewählt sind aus einem Wasserstoffatom und ($C_1$-$C_5$-Alkyl)gruppen und n eine ganze Zahl von 3 bis 10 ist.

2. Mittel nach Anspruch 1, wobei der Wirkstoff umfaßt (Cycloheptylamino)methylen-bis-(phosphonsäure) oder einen ($C_1$-$C_5$-Alkyl)ester oder ein Salz davon.

3. Die Knochenresorption hemmendes Mittel nach Anspruch 1 oder 2.

4. Antiarthritisches Mittel nach Anspruch 1 oder 2.

5. (Cycloalkylamino)methylen-bis-(phosphonsäure) oder -ester (I) oder ein Salz davon

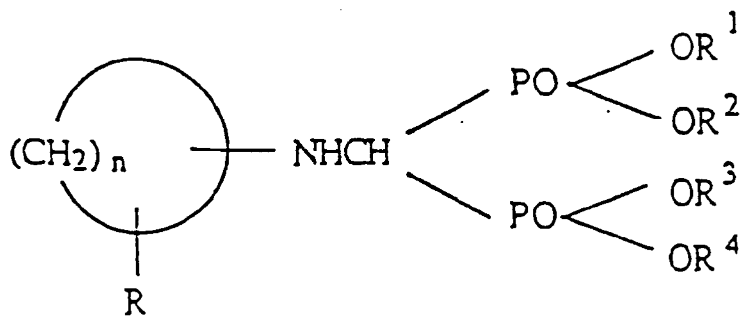

(I)

wobei R, $R^1$, $R^2$, $R^3$ und $R^4$ unabhängig voneinander ausgewählt sind aus einem Wasserstoffatom und ($C_1$-$C_5$-Alkyl)gruppen und n eine ganze Zahl von 3 bis 10 ist, mit der Maßgabe, daß R ($C_1$-$C_5$-Alkyl) ist, wenn $R^1$, $R^2$, $R^3$ und $R^4$ Wasserstoffatome sind und n 5 oder 6 ist.

6. Verbindung nach Anspruch 5, die (Cycloheptylamino)methylen-bis-(phosphonsäure) oder ein ($C_1$-$C_5$-Alkyl)ester oder ein Salz davon ist.

7. Verfahren zur Herstellung einer Verbindung nach Anspruch 5, umfassend die Umsetzung von (a) dem entsprechenden Cycloalkylamin, (b) phosphoriger Säure und/oder dem/den entsprechenden $C_1$-$C_5$-Alkylester(n) davon und (c) ($C_1$-$C_5$-Alkyl)orthoformiat, und gegebenenfalls Umwandlung des Produktes in die oder aus der Säure-, Ester- oder Salzform.

**Patentansprüche für folgende Vertragsstaaten : ES, GR**

1. Verfahren zur Herstellung einer Verbindung, die eine (Cycloalkylamino)methylen-bis-phosphonsäure oder ein -ester (I) oder ein Salz davon ist

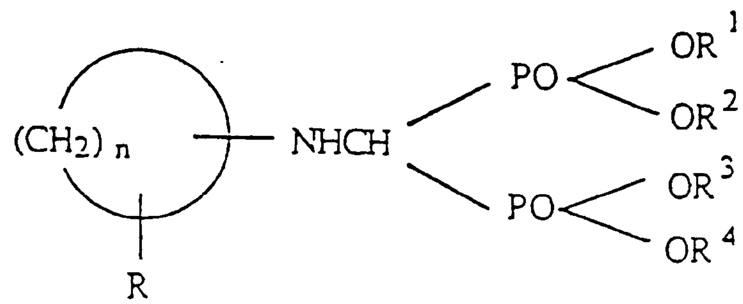

(I)

wobei R, $R^1$, $R^2$, $R^3$ und $R^4$ unabhängig voneinander ausgewählt sind aus einem Wasserstoffatom und ($C_1$-$C_5$-Alkyl)gruppen und n eine ganze Zahl von 3 bis 10 ist, mit der Maßgabe, daß R ($C_1$-$C_5$-Alkyl) ist, wenn $R^1$, $R^2$, $R^3$ und $R^4$ Wasserstoffatome sind und n 5 oder 6 ist, umfassend die Umsetzung von (a) dem entsprechenden Cycloalkylamin

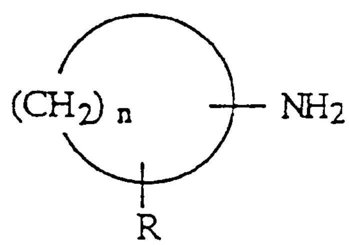

(b) phosphoriger Säure und/oder dem/den entsprechenden ($C_1$-$C_5$)-Alkylester(n) davon

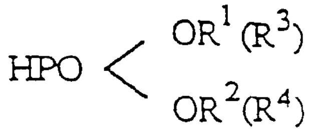

und (c) ($C_1$-$C_5$Alkyl)orthoformiat

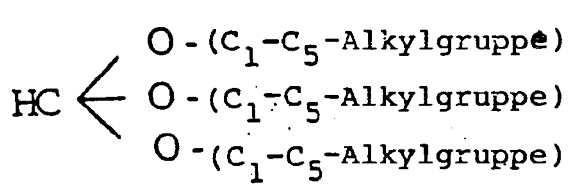

und gegebenenfalls Umwandlung des Produktes in die oder aus der Säure; Ester.oder Salzform.

2. Verfahren nach Anspruch 1, wobei das Produkt (Cycloheptylamino)methylen-bis-(phosphonsäure) oder ein ($C_1$-$C_5$-Alkyl)ester oder ein Salz davon ist.

## Revendications

### Revendications pour les Etats contractants suivants : AT, BE, CH, DE, FR, GB, IT, LI, LU, NL, SE

1. Une composition pharmaceutique contenant un principe actif choisi parmi les acides cycloalkylaminométhylènebisphosphoniques, et les esters (I) et les sels pharmaceutiquement acceptables de celui-ci.

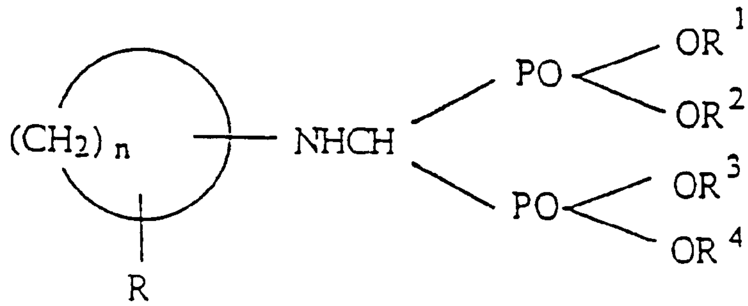

(I)

dans lequel R, $R^1$, $R^2$, $R^3$ et $R^4$ sont choisis de manière indépendante parmi un atome d'hydrogène et des groupements alcoyle en $C_1$-$C_5$, et n est un nombre entier de 3 à 10.

2. Une composition conforme à la revendication 1, dans laquelle le principe actif comprend un acide cycloalkylaminométhylènebisphosphonique, ou un ester d'alcoyle en $C_1$-$C_5$ ou un sel de celui-ci.

3. Une composition inhibitrice de la résorption osseuse conforme à l'une des revendications 1 ou 2.

4. Une composition anti-arthritique conforme à l'une des revendications 1 ou 2.

5. Un acide cycloalkylaminométhylènebisphosphonique, ou un ester (I) ou un sel de celui-ci.

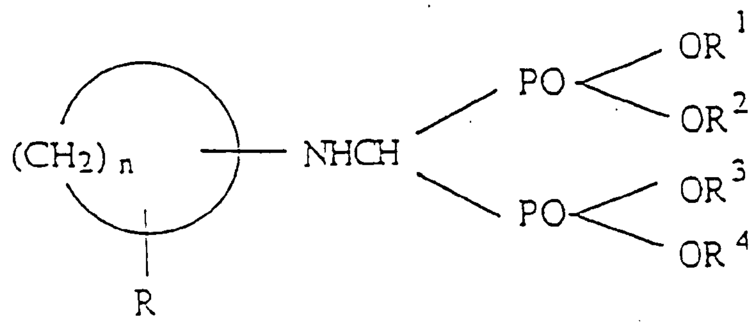

(I)

dans lequel R, $R^1$, $R^2$, $R^3$ et $R^4$ sont choisis de manière indépendante parmi un atome d'hydrogène et des groupements alcoyle en $C_1$-$C_5$, et n est un nombre entier de 3 à 10, à condition que R soit un groupement alcoyle en $C_1$-$C_5$ quand $R^1$, $R^2$, $R^3$ et $R^4$ sont des atomes d'hydrogène et n est égal à 5 ou 6.

6. Un composé conforme à la revendication 5, qui est l'acide cycloheptylaminométhylènebisphosphonique, ou un ester d'alcoyle en $C_1$-$C_5$ ou un sel de celui-ci.

7. Un procédé de préparation d'un composé tel que défini dans la revendication 5, qui consiste à faire réagir (a) la cycloalkylamine correspondante, (b) l'acide phosphorique et/ou un (des) ester(s) d'alcoyle en $C_1$-$C_5$ correspondant(s) de celui-ci, et (c) un orthoformiate d'alcoyle en $C_1$-$C_5$ et éventuellement transformer le produit en ou à partir de la forme acide, ester ou sel.

### Revendications pour les états contractants suivants : ES, GR

1. Un procédé de préparation d'un composé qui est un acide cycloalkylaminométhylènebisphosphonique, ou un ester (I) ou un sel de celui-ci

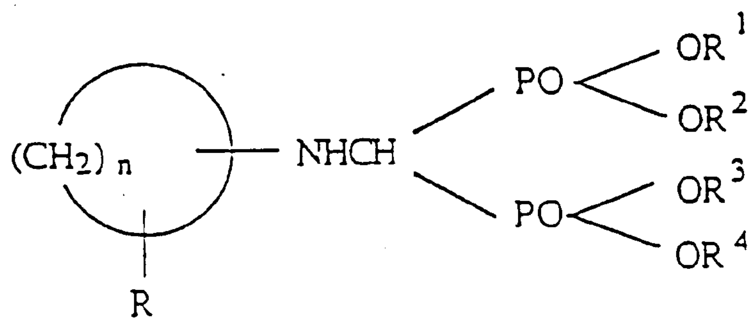

(I)

dans lequel R, $R^1$, $R^2$, $R^3$ et $R^4$ sont choisis de manière indépendante parmi un atome d'hydrogène et des groupements alcoyle en $C_1$-$C_5$, et n est un nombre entier de 3 à 10, à condition que R soit un groupement alcoyle en $C_1$-$C_5$ quand $R^1$, $R^2$, $R^3$ et $R^4$ sont des atomes d'hydrogène et n est égal à 5 ou 6, qui consiste à faire réagir (a) la cycloalkylamine correspondante

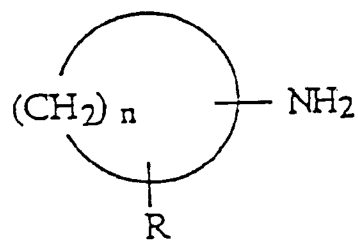

(b) l'acide phosphorique et/ou le (les) ester(s) d'alcoyle en $C_1$-$C_5$ correspondant(s) de celui-ci

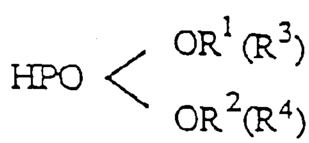

et (c) l'orthoformiate d'alcoyle en $C_1$-$C_5$

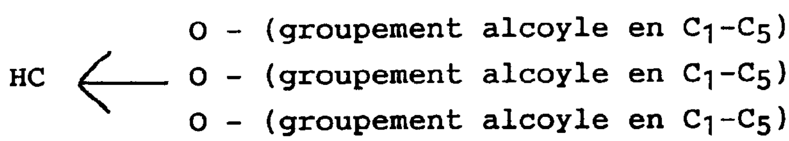

et éventuellement transformer le produit en ou à partir de la forme acide, ester ou sel.

2. Un procédé conforme à la revendication 1, dans lequel le produit est l'acide cycloheptylaminométhylènebisphosphonique, ou un ester d'alcoyle en $C_1$-$C_5$ ou un sel de celui-ci.